Europäisches Patentamt

European Patent Office

Office européen des brevets

(19)

(11) Veröffentlichungsnummer: **0 233 595**
**A2**

# (12) EUROPÄISCHE PATENTANMELDUNG

(21) Anmeldenummer: 87101963.4

(22) Anmeldetag: 12.02.87

(51) Int. Cl.4: **C12Q 1/34** , //G01N33/66

(30) Priorität: 21.02.86 DE 3605572

(43) Veröffentlichungstag der Anmeldung:
26.08.87 Patentblatt 87/35

(84) Benannte Vertragsstaaten:
AT BE CH DE ES FR GB GR IT LI LU NL SE

(71) Anmelder: **Boehringer Mannheim GmbH**
**Sandhofer Strasse 116**
**D-6800 Mannheim 31(DE)**

(72) Erfinder: **Kühnle, Hans-Frieder, Dr. rer. nat.**
**Silcher Weg 6**
**D-6940 Weinheim(DE)**
Erfinder: **von Dahl, Katharina**
**Schwarzwaldstrasse 27**
**D-6800 Mannheim 1(DE)**
Erfinder: **Schmidt, Felix Helmut, Prof.**
**Stockacher Strasse 1**
**D-6800 Mannheim 61(DE)**

(54) **Enzymatische Bestimmung von Inulin.**

(57) Gegenstand der Erfindung ist ein Verfahren zur Bestimmung von Inulin, bei dem Inulin enzymatisch zu Fructose umgesetzt, eventuell in der Probe vorhandene Glucose enzymatisch beseitigt und die freigesetzte Fructose in bekannter Weise enzymatisch bestimmt wird. Ferner wird ein Reagenz zur Durchführung des Verfahrens zur Bestimmung von Inulin beansprucht.

EP 0 233 595 A2

## Enzymatische Bestimmung von Inulin

Die Erfindung betrifft ein Verfahren zur Bestimmung von Inulin, bei dem Inulin enzymatisch zu Fructose umgesetzt und diese in bekannter Weise bestimmt wird, sowie ein hierfür geeignetes Reagenz.

Zur korrekten Ermittlung der renalen Eliminationsfunktionen werden heute vorzugsweise Radiopharmazeutika verwendet. Zur Bestimmung der glomerulären Clearance werden dabei solche markierten Verbindungen eingesetzt, die sich durch eine rein glomeruläre Filtration auszeichnen und weder tubulär sezerniert noch rückresorbiert werden. Die verwendeten Substanzen sollten weiterhin nicht extrarenal eliminiert werden und keine Bindung an Plasmaproteine eingehen. Für diese Untersuchungen haben sich vor allem Komplexbildner vom Typ der Ethylendiamin-tetra-essigsäure (EDTA) und Diethylentriamin-pentaessigsäure (DTPA) bewährt. Vor allem $^{52}$Cr-EDTA hat sich als besonders geeignet erwiesen.

In der allgemeinen nephrologischen Routine wird jedoch nach wie vor die Creatinin-Clearance bestimmt. Dazu muß die Creatinin-Konzentration im Blut sowie die im Verlauf einer zumeist 24stündigen Urinsammelperiode eliminierte Creatininmenge gemessen werden.

Die nuklearmedizinische Bestimmung der glomerulären und tubulären Filtrationsarten ist heute die Methode der Wahl. Die Durchführung dieser Untersuchungen ist allerdings mit relativ hohem apparativen Aufwand verbunden, so daß diese Bestimmungen nur an besonders dafür eingerichteten Zentren vorgenommen werden können. Darüber hinaus ist mit einer gewissen Strahlenbelastung zu rechnen, die vor allem dann ins Gewicht fällt, wenn zur Verlaufskontrolle wiederholte Untersuchungen durchgeführt werden müssen.

Die Creatinin-Clearance kann von jedem Praxislabor durchgeführt werden. Die Nachteile dieser Methode liegen jedoch vor allem in der oft unzuverlässig durchgeführten Urinsammelperiode sowie in physiologischen Schwankungen der Creatinin-Konzentration im Blut und in Schwankungen der tubulären Rückresorption begründet. Ein weiteres Problem stellt die relative Unspezifität der gebräuchlichen Creatinin-Bestimmungsmethoden dar.

Das klassische Material zur Bestimmung der glomerulären Clearance ist das Inulin. Da diese Substanz sich pharmakologisch weitgehend inert verhält, praktisch nicht an Plasmaprotein gebunden ist und ausschließlich glomerulär filtriert wird, ist sie für Clearance-Untersuchungen geeignet. Die genannten Vorteile werden allerdings dadurch relativiert, daß sich die bisher bekannten Inulin-Bestimmungsmethoden nicht nur durch einen hohen Arbeitsaufwand, sondern auch durch Ungenauigkeit und Unspezifität auszeichnen. Außerdem wird die praktische Durchführung der Bestimmung der Inulin-Clearance dadurch kompliziert, daß eine kontinuierliche Infusion der Substanz bis zum Erreichen einer steady state-Konzentration erforderlich ist. Anschließend muß die Inulin-Konzentration im Blut sowie die über einen definierten Zeitraum ausgeschiedene Inulinmenge im Harn gemessen werden. Dazu ist in der Regel eine Katheterisierung des Patienten unumgänglich [P. Kramer et al., Mitt. Klin. Nephrologie, 1, 26-59 (1974)].

Zur Bestimmung des Inulins in biologischen Proben sind verschiedene Verfahren bekannt. Sie beruhen üblicherweise auf der Bildung eines Farbstoffs, dessen Konzentration spektrophotometrisch bestimmt wird und ein Maß für die Inulin-Konzentration in der zu bestimmenden Probe darstellt. Bekannt sind solche Methoden, die mit Resorcin [G. F. Schreiner, Proc. Soc. Exp. Biol. Med., 74, 117 (1950)], Diphenylamin [H. D. Harrison, Proc. Soc. Exp. Biol. Med., 48 , 111 (1942)] oder Anthron [W. D. Davidson et al., J. Lab. Clin. Med., 62, 351 (1963)] arbeiten. Alle diese Methoden sind unspezifisch, beziehungsweise ungenau und müssen daher als veraltet angesehen werden.

Den genannten Bestimmungsverfahren ist gemeinsam, daß als erster Schritt das Polyfructosan Inulin einer sauren Hydrolyse unterzogen wird, die zumeist eine längere Reaktionszeit bei Temperaturen zwischen 37 und 75° C beansprucht. Diese Reaktionsbedingungen komplizieren die Probenbehandlung außerordentlich, da ein Flüssigkeitsverlust durch Verspritzen oder Verdampfen nicht ausgeschlossen werden kann. Gegebenenfalls muß in einem zugeschmolzenen Glaskolben gearbeitet werden.

Da die Hydrolyse bei Stark saurem pH-Wert durchgeführt wird, muß die Probe zuvor enteiweißt werden. Hierbei besteht die Gefahr, daß Inulin beim Ausfällen der Proteine mitgerissen und so die Bestimmung verfälscht wird.

Es bestand deshalb Bedarf an einer leicht durchzuführenden Inulinbestimmungsmethode. Die Aufgabe wird erfindungsgemäß dadurch gelöst, daß eine voll enzymatische Methode entwickelt wurde, die ohne Enteiweißung durchgeführt werden kann und die bei hoher Präzision und Richtigkeit mit kleinen Probenvolumina auskommt. Damit ist es möglich, die Inulin-Clearance mit hoher Genauigkeit analog den radiologischen Methoden zu bestimmen. Es wird ein Verfahren zur Verfügung gestellt, das ohne Schwierigkeiten von jedem -mit einem Photometer ausgerüsteten -Labor durchgeführt werden kann.

Gegenstand der Erfindung ist ein Verfahren zur Bestimmung von Inulin, bei dem Inulin enzymatisch zu Fructose umgesetzt, eventuell in der Probe vorhandene Glucose enzymatisch beseitigt und die freigesetzte Fructose in bekannter Weise enzymatisch bestimmt wird.

Zur Bestimmung des Inulins wird das Polyfructosan in seine Fructosebausteine gespalten. Der hydrolytische Abbau erfolgt enzymatisch. Als geeignete Enzyme können hierzu beispielsweise β-Fructosidase oder Inulinase verwendet werden. Für die Bestimmung von Fructose stehen dem Fachmann zahlreiche Methoden zur Verfügung. Zweckmäßigerweise wird die entstandene Fructose mit ATP durch Hexokinase phosphoryliert, Fructose-6-phosphat mit Glucose-6-phosphat-isomerase zu Glucose-6-phosphat isomerisiert und letzteres durch Glucose-6-phosphat-dehydrogenase mit NADP in Gluconat-6-phosphat und NADPH umgewandelt. Die Zunahme an NADPH wird gemessen. Sie ist ein Maß für die Konzentration des in der zu bestimmenden Probe vorliegenden Inulins.

Um die in den Serumproben stets in wechselnden Konzentrationen vorhandene Glucose nicht getrennt bestimmen zu müssen, kann sie mit Glucoseoxidase und Wasserstoffperoxid oxidiert werden. Sowohl die Glucoseoxidation als auch die enzymatische Hydrolyse des Inulins sind bei identischem pH-Wert und im gleichen Puffersystem durchzuführen. Beide Reaktionen können simultan nebeneinander ablaufen. Dies hat darüber hinaus den Vorteil, daß eventuell in der β-Fructosidasepräparation vorhandene Glucose mitoxidiert wird und somit die weitere Bestimmug nicht gestört wird.

Die erfindungsgemäße vollenzymatische Inulin-Bestimmungsmethode vermeidet die Nachteile der bekannten Verfahren des Standes der Tecknik. Sowohl Glucoseoxidation als auch enzymatische Hydrolyse des Inulins werden bei schwach saurem pH-Wert durchgeführt. Deshalb kann eine vorausgehende Enteiweißung der Probe entfallen. Durch die Oxidation der nativen Glucose wird außerdem eine Differenzanalyse vermieden. Dies trägt beträchtlich zur Genauigkeit der Methode bei, wenn man bedenkt, daß die Konzentration der nativen Glucose in Blutserum oder Plasma um den Faktor 100 oder mehr größer sein kann als die zu bestimmende Inulinkonzentration. Durch die nachgeschaltete enzymatische Bestimmung der Fructose wird schließlich eine außerordentlich hohe Spezifität erreicht.

In einer bevorzugten Ausführungsform des erfindungsgemäßen Verfahrens wird zunächst die Probe - beispielsweise Blutserum oder Plasma, gegebenenfalls mit physiologischer Kochsalzlösung verdünnt -mit einem geeigneten Puffersystem, pH 4,0 -6,0, vorzugsweise Citratpuffer, Glucoseoxidase und einem Inulin zu Fructose hydrolysierenden Enzym sowie mit Wasserstoffperoxid versetzt. Die Reaktionsmischung wird etwa 1 Stunde bei 37° C inkubiert. Die Bestimmung der aus Inulin durch enzymatische Hydrolyse erzeugten Fructose erfolgt anschließend bei einer Wellenlänge von 320 -370 nm. Hierzu wird in den Inkubationsansatz eine Lösung aus Magnesiumsalz, NADP und ATP in Triethanolaminpuffer gegeben. Es wird gut gemischt und die gesamte Mischung in eine Küvette überführt. An einem Spektrophotometer wird die Extinktion $E_1$ gemessen. Anschließend wird durch Zugabe einer Mischung der Enzyme Hexakinase, Glucose-6-phosphat-isomerase und Glucose-6-phosphat-dehydrogenase eine enzymatische Reaktionskette gestartet. Nach ca. 10 -20 Minuten ist die Reaktion abgeschlossen und es wird die Extinktion $E_2$ abgelesen. Aus der Extinktionsdifferenz läßt sich der Fructosegehalt und damit der Gehalt an Inulin in der Probe bestimmen. Es empfiehlt sich, neben der eigentlichen Bestimmung der Probe einen Reagenzienleerwert zu messen, welcher bei der Berechnung des Inulingehalts der Probe berücksichtigt werden muß.

Die für das erfindungsgemäße Verfahren benötigten Enzyme und Hilfsstoffe werden zweckmäßig zu bestimmten Lösungen beziehungsweise Suspensionen zusammengefaßt. Als Puffersystem wird vorzugsweise ein Citratpuffer mit pH 4,0 -6,0 in einer Konzentration von 0,05 -0,5 mol/l Pufferlösung, vorzugsweise 0,1 -0,3 mol/l Pufferlösung, eingesetzt. Ganz besonders bevorzugt ist ein Citratpuffer vom pH-Wert 5,0 der in einer Konzentration von 0,2 mol/l Pufferlösung bei der Bestimmung benutzt wird. Glucoseoxidase und ein Inulin zu Fructose hydrolysierendes Enzym werden vorteilhafterweise in einem der genannten Citratpuffer gelöst und als Inkubationsmedium I verwendet. Als Inulin zu Fructose hydrolysierende Enzyme können z. B. β-Fructosidase oder Inulinase eingesetzt werden. Die Konzentrationen der Enzyme in Inkubationsmedium I betragen 50 -300 U/ml, vorzugsweise 125 U/ml Glucoseoxidase und 0,5 -5 U/ml, vorzugsweise 0,75 U/ml Inulinase oder 2000 -10000 U/ml, vorzugsweise 6000 U/ml β-Fructosidase. Die Oxidation eventuell in der Probe vorhandener Glucose wird durch das Inkubationsmedium II gestartet, welches 0,1 -2 %, vorzugsweise 0,3 % Wasserstoffperoxid in Pufferlösung enthält.

Die für die Fructosebestimmung erforderlichen Hilfsstoffe wie NADP, ATP und Magnesiumsalz, z. B. Magnesiumsulfat können als Mischung in Triethanolaminpuffer aufbewahrt und eingesetzt werden.

Verwendbar ist z. B. ein 0,1 -2 mM, bevorzugterweise ein 0,3 mM Triethanolaminpuffer pH 6,0 -8,0. Besonders bevorzugt ist ein solcher Puffer mit pH 7,6.

Die Konzentrationen der darin gelösten Stoffe betragen für NADP 0,5 -3 mmol/l, vorzugsweise 1 mmol/l, für ATP 1,0 -5 mmol/l, vorzugsweise 2,5 mmol/1 und für ein Magnesiumsalz, wie z. B. Magnesiumsulfat 1,0 -10 mmol/l, vorzugsweise 4 mmol/l.

Die zur Bestimmung von Fructose erforderlichen Enzyme können vor der Bestimmung ebenfalls gemischt und als Mischung der Probe zugegeben werden. Vorteilhafterweise liegt diese Enzymmischung als Suspension in Ammoniumsulfatlösung vor. Die Konzentration der Ammoniumsulfatlösung beträgt üblicherweise 3 -4, vorzugsweise 3,2 mol/l. Sie besitzt einen pH-Wert von 5,5 - 6,5 vorzugsweise 6,0. Die Konzentrationen der Enzyme in der Mischung betragen 40 -400 U/ml, vorzugsweise 140 U/ml Hexokinase, 100 -1000 U/ml, vorzugsweise 350 U/ml Glucose-6-phosphat-isomerase und 20 -200 U/ml, vorzugsweise 70 U/ml Glucose-6-phosphat-dehydrogenase.

Gegenstand der Erfindung ist ferner ein Reagenz zur enzymatischen Bestimmung von Inulin durch dessen Hydrolyse zu Fructose, gegebenenfalls Zerstörung in der Probe vorhandene Glucose und Bestimmung der freigesetzten Fructose in bekannter Weise. Das Reagenz enthält die für das Verfahren zur Bestimmung von Fructose üblicherweise erforderlichen Stoffe sowie ein Inulin zu Fructose hydrolysierendes Enzym, wie Inulinase oder β-Fructosidase, sowie gegebenenfalls Glucoseoxidase und Wasserstoffperoxid. Ein im Sinne der Erfindung vorteilhaftes Reagenz enthält folgende Bestandteile:

a) eine Pufferlösung mit

0,05 -0,5 mol/l Citratpuffer, pH 4,0 -6,0

b) eine Enzymlösung mit

0,5 -5 U/ml Inulinase und gegebenenfalls

50 -300 U/ml Glucoseoxidase in Pufferlösung a)

c) gegebenenfalls eine

0,1 -2 %ige Wasserstoffperoxidlösung in Pufferlösung a)

d) eine Coenzymlösung mit

0,5 -3 mmol/l NADP

1,0 -5 mmol/l ATP und

1,0 -10 mmol/l Magnesiumsalz in

0,1 -2 mmol/l Triethanolaminpuffer, pH 6,0 -8,0

e) eine Enzymlösung mit

40 -400 U/ml Hexokinase,

100 -1000 U/ml Glucose-6-phosphat-isomerase und

20 -200 U/ml Glucose-6-phosphat-dehydrogenase.

Ganz besonders bevorzugt ist ein Reagenz mit folgenden Bestandteilen:

a) einer Pufferlösung mit

0,2 mol/l Citratpuffer, pH 5,0

b) einer Enzymlösung mit

0,75 U/ml Inulinase und gegebenenfalls

125 U/ml Glucoseoxidase in Pufferlösung a)

c) gegebenenfalls einer

0,3 %igen Wasserstoffperoxidlösung in Pufferlösung a)

d) einer Coenzymlösung mit

1 mmol/l NADP

2,5 mmol/l ATP

4,0 mmol/l Magnesiumsulfat in

0,3 mM Triethanolaminpuffer, pH 7,6,

e) einer Enzymlösung mit

140 U/ml Hexokinase,

350 U/ml Glucose-6-phosphat-isomerase und

70 U/ml Glucose-6-phosphat-dehydrogenase.

Statt Inulinase kann das Reagenz auch 2000 -10000 U/ml, vorzugsweise 6000 U/ml β-Fructosidase enthalten.

Die Erfindung wird durch das folgende Beispiel näher erläutert:

Beispiel 1

4

Bestimmung von Inulin

A) Herstellung der Lösungen

a) Citratpuffer 0,2 M, pH 5,0

4,2 g Zitronensäure ($C_6H_8O_7$ x $H_2O$) werden in Wasser ad 100 ml gelöst.
5,88 g Natrium-citrat ($C_6H_5Na_3O_7$ x 2 $H_2O$) werden in Wasser ad 100 ml gelöst.
60 ml Natrium-citrat-Lösung werden mit 25 ml der Zitronensäure-Lösung versetzt; die Mischung wird auf pH 5,0 eingestellt.

b) Inkubationsmedien

Inkubationsmedium I

Citratpuffer 0,2 M, pH 5,0 10,0 ml
Glucoseoxidase (ca. 250 U/mg) 5,0 mg
Inulinase (ca. 37 U/ml) 0,2 ml
Novozym SP 230, EC 3.2.1.7,
Novo Industrie, Bestell-Nr.811221
bzw. β-Fructosidase (ca. 300 U/mg) 200,0 mg
EC 3.2.1.26,
Boehringer Mannheim GmbH,
Bestell-Nr. 104914

Inkubationsmedium II

Citratpuffer 0,2 M, pH 5,0 5,0 ml
Wasserstoffperoxid, 30prozentige wässrige Lösung 0,05 ml

c) Reagenzien für die Fructosebestimmung

Lösung 1

Triethanolaminpuffer 0,3 mM, pH 7,6
$MgSO_4$ (4,0 mmol/l)
NADP (1,0 mmol/l)
ATP (2,5 mmol/l)

Lösung 2

Hexokinase (ca. 140 U/ml)
Glucose-6-phosphat-isomerase (ca. 350 U/ml)
Glucose-6-phosphat-dehydrogenase (ca. 70 U/ml)
als Suspension in Ammoniumsulfatlösung 3,2 mol/l, pH 6,0

B) Durchführung der Bestimmung

a) Probevolumina

Bei Humanversuchen kann mit einem einheitlichen Probenvolumen von 0,1 ml Serum oder Plasma gearbeitet werden. Damit kann z. B. die Eliminationskinetik des Inulins nach intravenöser Applikation von 5 g pro Versuchsperson bis zu einem Zeitraum von ca. 4 Stunden post applicationem sicher erfaßt werden. Die Serumkonzentration bewegt sich dabei bei nierengesunden Probanden in einem Bereich von ca. 1 mg/ml bis 0,09 mg/ml.

Bei Untersuchungen mit kleineren Tierspezies muß im allgemeinen mit einer relativ höheren Inulindosis gearbeitet werden. Daher empfiehlt es sich, um den Meßbereich nicht zu überschreiten, die Proben während der Verteilungsphase (bis ca. 30 min post applicationem) mit physiologischer Kochsalzlösung auf 1:10 zu verdünnen. Während der linearen Eliminationsphase, die relativ rasch zu niedrigen Konzentrationen führt, sollte die Probe dagegen unverdünnt eingesetzt werden.

b) Oxidation und Hydrolyse

Reaktionsansatz:

| | Humanversuche | Tierversuche |
|---|---|---|
| Probe | 0,1 ml | 0,05 ml |
| Inkubationsmedium I | 0,2 ml | 0,10 ml |
| Inkubationsmedium II | 0,1 ml | 0,05 ml |

Die Reaktionsansätze werden gemischt und ca. 60 min bei 37° C inkubiert.

c) Bestimmung der Fructose

Die Bestimmung erfolgt bei einer Wellenlänge von 334 nm.
In den Inkubationsansatz wird pipettiert:
Lösung 1 1,0 ml
Es wird gut gemischt und die gesamte Mischung wird in eine Halbmikroküvette überführt.
E 1 wird abgelesen.

Zugabe des Enzymgemischs

Lösung 2 0,02 ml
Es wird gemischt und das Reaktionsende (ca. 10 -20 min) abgewartet.
E 2 wird abgelesen.
Ist der Reagenzienleerwert nicht zu vernachlässigen, so muß er in der Berechnung berücksichtigt werden.

$E_1 - E_2 - \Delta E_{Leerwert} = \Delta E_{Fructose}$

C) Berechnung

a) Humanproben

Probevolumen 0,1 ml
Küvettenvolumen 1,42 ml

$$\Delta E_{Fructose} \times 0,665 = mg\ Inulin/ml$$

b) Tierproben

Probevolumen 0,05 ml
Küvettenvolumen 1,22 ml
$$\Delta E_{Fructose} \times 1,143 = mg\ Inulin/ml$$
Bei den verdünnten Proben muß das Ergebnis mit 10 multipliziert werden.

D) Ergebnisse

a) Präzision

In Tabelle 1 sind die Mittelwerte und die Streuungsmaße von je 10 Inulinbestimmungen mit Inulinase bzw. β-Fructosidase aus wässriger Lösung oder gepooltem Kaninchenserum aufgelistet. Der Variationskoeffizient liegt bei der Verwendung von β-Fructosidase bei Werten um ≤ 3 %; beim Einsatz von Inulinase als hydrolysierendem Enzym konnten Variationskoeffizienten von ≤ 1 % ermittelt werden.

## Tabelle 1
### Präzision der Inulin-Bestimmung mit Inulinase bzw. ß-Fructosidase in wässriger Lösung und in gepooltem Kaninchen-Serum

|  | wässrige Lösung | | Kaninchen-Serum | |
| --- | --- | --- | --- | --- |
|  | Inulinase | ß-Fructosidase | Inulinase | ß-Fructosidase |
| N | 10 | 10 | 10 | 10 |
| Mittelwert (mg/ml) | 0,49 | 0,46 | 0,49 | 0,46 |
| SDV | 0,005 | 0,014 | 0,004 | 0,008 |
| SEM | 0,002 | 0,004 | 0,001 | 0,002 |
| VK | 1,03 | 3,04 | 0,82 | 1,74 |

N   = Zahl der durchgeführten Bestimmungen
SDV = Standardabweichung
VK  = Variationskoeffizient
SEM = Mittelwertsfehler

b) Wiederfindung

In Tabelle 2 sind die Meßergebnisse der Inulinbestimmung bei verschiedenen Substratkonentrationen unter Verwendung von Inulinase bzw. β-Fructosidase in wässriger Lösung und gepooltem Kaninchenserum dargestellt.

Die dokumentierten Werte zeigen eine ausgezeichnete Wiederfindungsrate in allen Konzentrationsbereichen.

0 233 595

## Tabelle 2

Ergebnisse der Inulin-Bestimmung mit Inulinase bzw. ß-Fructosidase in wässriger Lösung und gepooltem Kaninchen-Serum bei verschiedenen Konzentrationen. (Mittelwerte von je 10 Bestimmungen)

| Eingesetzt | Gefunden | | | |
|---|---|---|---|---|
| Inulin | wässrige Lösung | | Kaninchen-Serum | |
| (mg/ml) | Inulinase | ß-Fructosidase | Inulinase | ß-Fructosidase |
| 0,05 | 0,05 | 0,05 | 0,06 | 0,05 |
| 0,1 | 0,10 | 0,10 | 0,10 | 0,10 |
| 0,2 | 0,20 | 0,19 | 0,21 | 0,20 |
| 0,5 | 0,49 | 0,46 | 0,49 | 0,46 |
| 1,0 | 0,96 | 0,92 | 0,99 | 1,00 |

**Ansprüche**

1. Verfahren zur Bestimmung von Inulin, dadurch gekennzeichnet, daß Inulin enzymatisch zu Fructose umgesetzt, eventuell in der Probe vorhandene Glucose enzymatisch beseitigt und die freigesetzte Fructose in bekannter Weise enzymatisch bestimmt wird.

2. Verfahren gemäß Anspruch 1, dadurch gekennzeichnet, daß die enzymatische Umsetzung von Inulin zu Fructose mittels Inulinase oder $\beta$-Fructosidase erfolgt.

3. Verfahren gemäß Anspruch 1 oder 2, dadurch gekennzeichnet, daß Fructose bestimmt wird, in dem sie durch Hexokinase mit ATP in Fructose-6-phosphat überführt, dieses durch Glucose-6-phosphat-isomerase zu Glucose-6-phosphat isomerisiert, letzteres durch Glucose-6-phosphat-dehydrogenase mit NADP in Gluconat-6-phosphat und NADPH umgewandelt und die Zunahme an NADPH gemessen wird.

4. Verfahren gemäß einem der Ansprüche 1 bis 3, dadurch gekennzeichnet, daß eventuell in der Probe vorhandene Glucose durch Glucoseoxidase und $H_2O_2$ beseitigt wird.

5. Reagenz zur Bestimmung von Inulin, dadurch gekennzeichnet, daß es ein Inulin zu Fructose hydrolysierendes Enzym, die zur Bestimmung von Fructose erforderlichen Enzyme, Coenzyme und Hilfsstoffe sowie gegebenenfalls Glucoseoxidase und Wasserstoffperoxid enthält.

6. Reagenz gemäß Anspruch 5, dadurch gekennzeichnet, daß es als ein Inulin zu Fructose hydrolysierendes Enzym Inulinase oder $\beta$-Fructosidase enthält.

7. Reagenz gemäß Anspruch 5 oder 6, dadurch gekennzeichnet, daß es folgende Bestandteile enthält:
   a) eine Pufferlösung mit
0,05 bis 0,5 mol/l Citratpuffer, pH 4,0 bis 6,0
   b) eine Enzymlösung mit
0,5 -5 U/ml Inulinase
und gegebenenfalls
50 bis 300 U/ml Glucoseoxidase in Pufferlösung a)
   c) gegebenenfalls eine
0,1 bis 2 %ige Wasserstoffperoxidlösung in Pufferlösung a)
   d) eine Coenzymlösung mit
0,5 -3 mmol/l NADP
1,0 -5 mmol/l ATP
1,0 -10 mmol/l Magnesiumsalz in
0,1 - 2 mmol/l Triethanolaminpuffer, pH 6,0 -8,0
   e) eine Enzymlösung mit
40 bis 400 U/ml Hexokinase
100 bis 1000 U/ml Glucose-6-phosphat-isomerase und
20 bis 200 U/ml Glucose-6-phosphat-dehydrogenase

8

8. Reagenz gemäß Anspruch 7, dadurch gekennzeichnet, daß es statt Lösung b) eine Enzymlösung b') mit

2000 bis 10000 U/ml β-Fructosidase und gegebenenfalls

50 bis 300 U/ml Glucoseoxidase in Pufferlösung a) enthält.

9. Reagenz gemäß Anspruch 5 und 6, dadurch gekennzeichnet, daß es folgende Bestandteile enthält:

a) eine Pufferlösung mit

0,2 mol/l Citratpuffer, pH 5,0

b) eine Enzymlösung mit

0,75 U/ml Inulinase und gegebenenfalls

125 U/ml Glucoseoxidase in Pufferlösung a)

c) gegebenenfalls eine

0,3 %ige Wasserstoffperoxidlösung in Pufferlösung a)

d) eine Coenzymlösung mit

1 mmol/l NADP

2,5 mmol/l ATP

4,0 mmol/l Magnesiumsulfat in

0,3 mM Triethanolaminpuffer, pH 7,6

e) eine Enzymlösung mit

140 U/ml Hexokinase

350 U/ml Glucose-6-phosphat-isomerase und

70 U/ml Glucose-6-phosphat-dehydrogenase

10. Reagenz gemäß Anspruch 9, dadurch gekennzeichnet, daß es statt Lösung b) eine Enzymlösung b') mit

6000 U/ml β-Fructosidase und gegebenenfalls

125 U/ml Glucoseoxidase in Pufferlösung a) enthält.